# EUROPEAN PATENT APPLICATION

(11) **EP 2 103 213 A1**
(43) Date of publication of application: **23.09.2009**
(21) Application number: 07859998.2
(22) Date of filing: 18.12.2007
(51) Int. Cl.: A01N 43/16, A01P 1/00, A23L 3/3544, A61K 31/351, A61P 31/04

(54) **ANTIBACTERIAL AGENT AND BACTERICIDAL AGENT**

(30) Priority: 22.12.2006 JP 2006346299
(71) Applicant: Kabushiki Kaisha Toyota Jidoshokki, Kariya-shi, Aichi 448-8671 (JP); J-Chemical, Inc., Tokyo 104-0044 (JP)
(72) Inventor: SHIGEHARA, Kiyotaka, Fuchu-shi Tokyo 183-8538 (JP); BITO, Masami, Tokyo 104-0044 (JP); KATAYAMA, Yoshihiro, Fuchu-shi Tokyo 183-8538 (JP)
(74) Representative: TBK-Patent
(86) International application number: PCT/JP2007/074769
(87) International publication number: WO 2008/078723

(57) **Abstract**

An antimicrobial agent comprising, as an active component, compounds represented by the following formula (I): 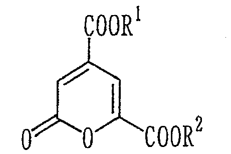 [wherein, R¹ and R² each independently represent H or an alkali metal atom.]; and a microbicidal agent comprising, as an active component, compounds represented by formula (I): 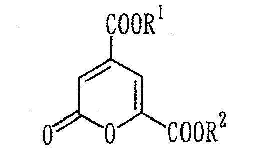 [wherein, R¹ and R² each independently represent H or an alkali metal atom.],
wherein they have high safety and little impact on the environment.

## Description

### FIELD OF THE INVENTION

The present invention relates to an antimicrobial agent and a microbicidal agent, having a good antimicrobial activity and a good microbicidal activity, and exhibiting high safety with little impact on the environment.

### BACKGROUND OF THE INVENTION

An enormous number of antimicrobial agents have been developed so far, and are used in all fields. The antimicrobial agents are classified roughly into inorganic antimicrobial agents such as silver or ceramics, and organic antimicrobial agents such as sorbic acid, benzoic acid, or salts thereof. The organic antimicrobial agents are produced using petroleum naphtha, as raw materials. Paraben, benzoic acid, salicylic acid, triclosan, and others are known as organic antimicrobial agents in the fields of cosmetics or food. Many of antimicrobial agents and antiseptics have problems with safety, and presently subjects to which the antimicrobial agents and antiseptics are allowed to be added, and the additive amounts thereof are limited.

An antimicrobial agent referred to as BIO-Add, which is used for producing animal feed, in order to inhibit unwanted microbes, such as bacteria (for example, Salmonella), yeasts, or molds, contains formic acid of 68%. However, formic acid per se is highly toxic and has problems with safety.

Natural antimicrobial agents with high safety are known, but also have problems that there are not many natural antimicrobial agents. In addition, for example, wasabi-thiol, etc., is expensive. The impact on the environment when the antimicrobial agents are discharged into nature, also must be considered.

As natural antimicrobial agents, antimicrobial agents derived from citrus are known (Japanese Unexamined Patent Publication (Kokai) No.8-317782). Such citrus-derived antimicrobial agents contain, as an active component, a compound obtained by purifying the residue which is a concentrate of essential oil obtained from a squeezed lemon peel. In order to obtain the desired antimicrobial agent, it is necessary to adjust the concentration thereof, and consequently, the manufacturing method becomes complicated and expensive.

An antimicrobial agent containing an extract from false indigo of Pea family is disclosed (Japanese Unexamined Patent Publication (Kokai) No. 2004-256437). However, false indigo is not a plant for cultivation and, not economical because of the need for solvent extraction.

An antimicrobial agent described in Japanese Unexamined Patent Publication (Kokai) No. 2002-161075 is produced from, as a raw material, gallic acid derived from tannin similar to the present invention, and synthesis thereof requires organic solvents, and highly toxic substances, such as a crown ether.

Further, an ester compound of ferulic acid produced from a natural ferulic acid, as an active component, is disclosed (Japanese Unexamined Patent Publication (Kokai) No.2002-161004), but ferulic acid derived from rice bran, per se, is expensive and the safety of the ester compound thereof has not been confirmed.

### DISCLOSURE OF THE INVENTION

An object of the present invention is to solve the above problems, and to provide antimicrobial agents and microbicidal agents, with high safety and little impact on the environment.

As the result of earnest investigation to attain the above-described object, the present inventors have found that 2H-pyran-2-on-4,6-dicarbonic acid (hereinafter referred to as "PDC") or alkali metal salts thereof represented by the following formula (I), have an antimicrobial activity and a microbicidal activity on Gram-negative bacteria and Gram-positive bacteria, and have accomplished the present invention.

That is, the present invention provides antimicrobial agents comprising, as an active component, compounds represented by formula (I): 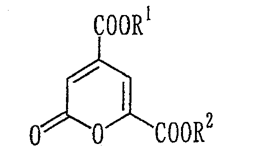 [wherein, R¹ and R² each independently represent H or an alkali metal atom.]

The present invention also provides microbicidal agents comprising, as an active component, compounds represented by formula (I): 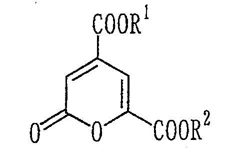 [wherein, R¹ and R² each independently represent H or an alkali metal atom.]

According to the present invention, PDC or alkali metal salts thereof have a good antimicrobial activity and microbicidal activity.

Therefore, PDC or alkali metal salts thereof, which are derived from nature, are industrially useful as the components of antimicrobial agents and microbicidal agents which have high safety with little impact on the environment.

### BEST MODE FOR CARRYING OUT THE INVENTION

Antimicrobial agents and microbicidal agents of the present invention comprise, as an active component, PDC or alkali metal salts thereof. As an alkali metal atom, lithium, sodium, potassium, and others can be used. The method for producing PDC is not limited to a specific method, and, for example, a method for cultivating cells transformed by introducing pKTVLABC which is a plasmid carrying out fermentative production of PDC, into Pseudomonas putida PpY1100, using vanillin, syringaldehyde, vanillic acid, syringic acid, or protocatechuic acid, as starting materials (Japanese Kokai No. 2005-278549), can be used. Alkali metal salts of PDC, by the method of production described in Japanese Unexamined Patent Publication (Kokai) No. 2005-278549, can be obtained at high-purity and good yield in the presence of a salt, such as sodium chloride, in a microbial fermentation broth containing PDC.

The amount of alkali metal salts of PDC represented by formula (I) in the antimicrobial agents and microbicidal agents of the present invention, is usually 0.1 to 20 % by weight, preferably 0.1 to 10 % by weight, more preferably 0.1 to 5 % by weight.

Antimicrobial agents and microbicidal agents of the present invention, have a good antimicrobial activity and microbicidal activity against various bacteria, which can be, for example, gram-positive bacteria, such as Staphylococcus aureus, Bacillus subtilis, coagulase-negative Staphylococcus, Streptococcus, or Eenterococcus; gram-negative bacteria, such as Escherichia coli, Shigella, Salmonella enterica, or Yersinia pestis.

Antimicrobial agents and microbicidal agents of the present invention, can be added into foods, drinks, cosmetics, oral care products, drugs, quasi drugs, and others, in order to, for example, provide a microbicidal effect on skin, that is, decolonization and disinfection by application to the body surface, or an antimicrobial effect, that is, inhibition of undesirable bacterial growth on the surface of teeth and in the mouth. In the preparation of oral care products, drugs, and others in which antimicrobial agents and microbicidal agents of the present invention are added, it is possible to blend components commonly used for oral care products and drugs within a range which does not impair the effects of the present invention.

### EXAMPLES

The invention will be explained below with reference to the examples, but the invention is not limited thereto.

### Example 1

### [Materials]

### (1) Test compound

PDC (R=H in formula (I)) (Purity: 99 %, Form:
powder, Storage condition: room temperature)

PDC was prepared by the methods according to Japanese Unexamined Patent Publication (Kokai) No. 2005-278549.

### (2) Preparation of 10 %, 1.0 % and 0.1 % aqueous solutions of the test compound

### (2-1) 10 % aqueous solution

1 g of the test compound was weighed and adjusted to 10 mL in volume by adding sterile purified water defined in Japanese Pharmacopoeia (manufactured by NIPPON SHINYAKU CO.,LTD, autoclaving treatment).

### (2-2) 1.0 % aqueous solution

10 % aqueous solution was diluted ten-fold with sterile purified water.

### (2-3) 0.1 % aqueous solution

10 % aqueous solution was diluted 100-fold with sterile purified water.

### (3) Strain

Staphylococcus aureus subsp. aureus (NBRC 13276) Escherichia coli (NBRC 3972) Salmonella Enteritidis (NBRC 3313)

Texts in parentheses represent the standard strain number.

### (4) Medium

Trypticase-Soy agar (SCD medium, manufactured by Japan BIOMERIEUX)

### (5) Sterile dish

Sterile NE dish (manufactured by Eiken kizai Co., Ltd.)

### [Test for antimicrobial activity]

After said strains were cultured in SCD agar medium at 35 ± 2°C for 18 to 24 hours, inocula were prepared by suspending the cultured stains in sterile purified water and adjusting the turbidity so as to correspond with No.1 MacFarland. Using agar plates, the concentrations of the inocula were measured according to the agar plate mixed dilution method (Table 1).

**Table 1**

| Inoculum strain | Concentration (cfu/mL) |
|---|---|
| Staphylococcus aureus subsp. aureus | 14 x 10⁷ |
| Escherichia coli | 37 x 10⁷ |
| Salmonella Enteritidis | 22 x 10⁷ |

3 mL of each of the 10 %, 1.0 % and 0.1 % aqueous solutions of the test compound, and of sterile purified water (control) were prepared (n=1). 0.03 mL of said inocula was inoculated into each of the test solutions at room temperature. Immediately after, 15 minutes after, and 180 minutes after the inoculations, 0.5 mL of each of the test solutions was added to 49.5 mL of a sterile phosphate buffer (Pro-media MV2-1000, manufactured by ELMEX), and mixed. A ten fold dilution series of each of the mixtures was constructed. Then, the number of the viable cells therein were measured, and then it was converted to the number of viable cells per 1 mL of the test solutions or control. The number of viable cell was measured according to "Viable cell count; the agar plate mixed dilution method in the Japanese Pharmacopoeia, 14th revised edition". The results are shown in Table 2.

**Table 2**

| (cfu/mL) | | | | | | | | | |
|---|---|---|---|---|---|---|---|---|---|
| | Staphylococcus aureus subsp. aureus | | | Escherichia coli | | | Salmonella Enteritidis | | |
| | Measured immediately after inoculation | Measured 15 min. after inoculation | Measured 180 min. after inoculation | Measured immediately after inoculation | Measured 15 min. after inoculation | Measured 180 min. after inoculation | Measured immediately after inoculation | Measured 15 min. after inoculation | Measured 180 min. after inoculation |
| 10 % of aqueous solution of the test compound | < 100 | < 100 | < 100 | < 100 | < 100 | < 100 | < 100 | < 100 | < 100 |
| 1.0 % of aqueous solution of the test compound | 5,000 | < 100 | < 100 | < 100 | < 100 | < 100 | < 100 | < 100 | < 100 |
| 0.1% of aqueous solution of the test compound | 11,000 | < 100 | <100 | 10,000 | < 100 | < 100 | 12,000 | < 100 | < 100 |
| Control sterile purified water | 27,000 | 27,000 | 22,000 | 18,000 | 12,000 | 19,000 | 13,000 | 15,000 | 14,000 |

As is obvious from Table 2, it has been shown that antimicrobial agents and microbicidal agents of the present invention inhibit the growth of the bacteria even at the lowest concentration of 0.1 % by weight.

### Example 2

### [Materials]

### (1) Test compound

PDC (R=H in formula (I)) (Purity: 99 %, Form: powder, Storage condition: room temperature)

PDC was prepared by the methods according to Japanese Unexamined Patent Publication (Kokai) No. 2005-278549.

### (2) Preparation of 20 % aqueous solution of the test compound

2 g of the test compound was weighed and adjusted to 10 mL in volume by adding sterile purified water defined in Japanese Pharmacopoeia (manufactured by NIPPON SHINYAKU CO., LTD, autoclaving treatment).

### (3) Comparative compound

Bio-Add (composition: formic acid 68%, propionic acid 20%, water 12%, Form: aqueous solution, Storage condition: room temperature, manufactured by Trouw Nutrition International).

### (4) Strain

Staphylococcus aureus subsp. aureus (NBRC 13276) Escherichia coli (NBRC 3972) Salmonella Enteritidis (NBRC 3313) Texts in parentheses represent the standard strain number.

### (5) Medium

Trypticase-Soy agar (SCD medium, manufactured by Japan BIOMERIEUX)
SCDLP Broth (manufactured by Eiken Chemical Co., Ltd.)

### (6) Sterile dish

Sterile NE dish (manufactured by Eiken kizai Co., Ltd.)

### [Test for antimicrobial activity]

After said strains were cultured in SCD agar medium at 35 ± 2°C for 18 to 24 hours, inocula were prepared by suspending the stains in sterile purified water and adjusting the turbidity so as to correspond with No.1 MacFarland. Using agar plate, the concentrations of the inocula were measured according to the agar plate mixed dilution method (Table 3).

**Table 3**

| Inoculum strain | Concentration (cfu/mL) |
|---|---|
| Staphylococcus aureus subsp. aureus | 20 x 10⁷ |
| Escherichia coli | 26 x 10⁷ |
| Salmonella Enteritidis | 46 x 10⁷ |

3 mL of each of 20 % aqueous solution of the test compound, a stock solution of a comparative compound and sterile water, (control) was prepared (n=1). 0.03 mL of said inocula was inoculated into each of the test solutions at a room temperature. Immediately after, 15 minutes after and 180 minutes after the inoculations, neutralization was performed under the following conditions.

### Test compound:

0.5 mL of the test solution was added to 4.5 mL of sterile sodium hydrate solution at the concentration of 0.25 mol/L (sodium hydrate; manufactured by Wako Pure Chemical Industries, Ltd.), and mixed. Then, 45 mL of a sterile phosphate buffer (Pro-media MV2-1000, manufactured by ELMEX) was immediately added and mixed.

### Comparative compound:

0.5 mL of the test solution was added to 4.5 mL of sterile sodium hydrate solution at the concentration of 2.0 mol/L, and mixed. Then, 45 mL of a sterile phosphate buffer was immediately added and mixed. Sterile water (control):

0.5 mL of the test solution was added to 49.5 mL of a sterile phosphate buffer and mixed.

A ten-fold dilution series of each of the neutralization solution was prepared. Then, the number of the viable cells therein was measured and it was converted to the number of viable cells per 1 mL of the test solutions or control. The number of viable cell was measured according to "Viable cell count; the agar plate mixed dilution method in the Japanese Pharmacopoeia, 14th revised edition". The results are shown in Table 4.

**Table 4**

| (cfu/mL) | | | | | | | | | |
|---|---|---|---|---|---|---|---|---|---|
| | Staphylococcus aureus subsp. aureus | | | Escherichia coli | | | Salmonella Enteritidis | | |
| | Measured immediately after inoculation | Measured 15 min. after inoculation | Measured 180 min. after inoculation | Measured immediately after inoculation | Measured 15 min. after inoculation | Measured 180 min. after inoculation | Measured immediately after inoculation | Measured 15 min. after inoculation | Measured 180 min. after inoculation |
| 20 % of aqueous solution of the test compound | < 100 | < 100 | < 100 | < 100 | < 100 | < 100 | < 100 | < 100 | < 100 |
| Comparative compound | < 100 | < 100 | < 100 | < 100 | < 100 | < 100 | < 100 | < 100 | < 100 |
| Control sterile purified water | 1,500,000 | 1,300,000 | 1,200,000 | 1,900,000 | 1,500,000 | 1,400,000 | 4,100,000 | 3,300,000 | 3,200,000 |

As is obvious from Table 4, antimicrobial agents and microbicidal agents of the present invention exhibited an antimicrobial activity and a microbicidal activity to the same extent as the comparative compound, BIO-Add.

### INDUSTORY APPLICABILITY

PDC or alkali salts thereof are used for the manufacturing of antimicrobial agents and microbicidal agents, which have high safety and little impact on the environment.

## Claims

1. An antimicrobial agent comprising, as an active component, compounds represented by formula (I): 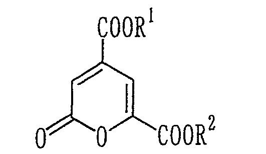 [wherein, R¹ and R² each independently represent H or an alkali metal atom.]

2. A microbicidal agent comprising, as an active component, compounds represented by formula (I): 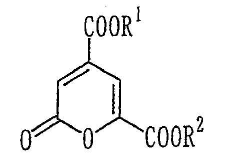 [wherein, R¹ and R² each independently represent H or an alkali metal atom.]
